# EUROPEAN PATENT APPLICATION

(11) **EP 4 772 115 A1**
(43) Date of publication of application: **08.07.2026**
(21) Application number: 24865669.6
(22) Date of filing: 25.07.2024
(51) Int. Cl.: A61B 8/00

(54) **ULTRASONIC PROBE**

(30) Priority: 13.09.2023 KR 20230121689
(71) Applicant: Alpinion Medical Systems Co., Ltd., Hwaseong-si, Gyeonggi-do 18365 (KR)
(72) Inventor: LEE, Won Seok, Seoul 07789 (KR); LEE, Sang Gon, Seoul 07789 (KR); LEE, Hyung Keun, Seoul 07789 (KR); WOO, Jeong Dong, Seoul 07789 (KR)
(74) Representative: Sander, Rolf
(86) International application number: PCT/KR2024/010788
(87) International publication number: WO 2025/058226

(57) **Abstract**

An ultrasonic probe having a sono-stair structure is disclosed. An ultrasonic probe according to an embodiment comprises: an active element having a thickness corresponding to a 1/4 wavelength to a 2/3 wavelength based on the center frequency of the ultrasonic probe; a matching layer formed on the active element; a grinding relief layer formed beneath the active element to separate a channel during a grinding process of the active element; and a sono-stair layer formed beneath the grinding relief layer and comprising a copper sheet; and a support formed beneath the sono-stair layer.

## Description

### [Technical Field]

The present invention relates to an ultrasonic probe that acquires image information of the interior of a target object by using ultrasound.

### [Background Art]

An ultrasonic diagnostic apparatus is an apparatus that irradiates ultrasonic signals to a target object and images internal tissue of the target object using ultrasonic signals reflected therefrom. The ultrasonic diagnostic apparatus may acquire image information of a diagnostic site by transmitting ultrasonic signals to the diagnostic site of the target object and then receiving ultrasonic signals reflected from a boundary between tissues inside the target object having different acoustic impedances.

The ultrasonic diagnostic apparatus includes an ultrasonic probe for transmitting ultrasonic signals to the target object and receiving ultrasonic signals reflected from the target object. A typical ultrasonic probe generally includes an active element, a matching layer, and a backing layer.

### [Disclosure]

### [Technical problem]

According to an embodiment, proposed is an ultrasonic probe having a sono-stair structure in which a channel separation process is improved and uniformity is enhanced through the generation of periodic and uniform back-reflected waves.

### [Technical Solution]

An ultrasonic probe according to an embodiment includes an active element having a thickness corresponding to 1/4 wavelength to 2/3 wavelength based on a center frequency of the ultrasonic probe, a matching layer formed above the active element, a sono-stair layer comprising a dicing buffer layer formed below the active element to separate channels during a dicing process of the active element and a copper sheet formed below the dicing buffer, and a support formed below the sono-stair layer.

The active element may have a thickness corresponding to 1/2 wavelength based on the center frequency of the ultrasonic probe.

The dicing buffer layer may have a thickness corresponding to 1/5 wavelength to 1/4 wavelength based on the center frequency of the ultrasonic probe. The dicing buffer layer may have a thickness corresponding to 1/4 wavelength based on the center frequency of the ultrasonic probe.

The dicing buffer layer may be made of an epoxy material.

The copper sheet may have a thickness corresponding to 1/20 wavelength or more based on the center frequency of the ultrasonic probe.

The sono-stair layer may further include a flexible printed circuit board formed between the active component and the dicing buffer layer to enable electrical connection of a plurality of separated channels of the active component.

The flexible printed circuit board may include a first flexible printed circuit board formed between the active element and a second flexible printed circuit board, and the second flexible printed circuit board formed between the first flexible printed circuit board and the dicing buffer layer.

The first flexible printed circuit board may include copper (Cu), and the second flexible printed circuit board may include polyimide (PI).

The ultrasonic probe may further include a ground sheet formed between the active element and the matching layer.

The ultrasonic probe may further include a first kerf formed extending from an upper surface of the matching layer through the active element to a first position within the dicing buffer layer.

The ultrasonic probe may further include a second kerf formed only in the active element, wherein the second kerf may be formed along a direction intersecting the first kerf.

The matching layer may include a plurality of layers each having a different acoustic impedance magnitude, the plurality of layers being formed using N materials (N is an integer greater than or equal to 2), to match acoustic impedance between the active element and a target object.

### [Advantages and Effects]

An ultrasonic probe according to an embodiment has an active element having a thickness corresponding to 1/4 wavelength to 2/3 wavelength (1/4 λ to 2/3λ) based on a center frequency of the ultrasonic probe. Accordingly, the ultrasonic probe is applicable to high frequencies. In addition, the ultrasonic probe has a wide allowable range of driving voltage, fabrication of a dicing buffer layer is easy, and manufacturing cost is low.

Also, in a conventional ultrasonic probe, since a backing layer is required to be cut during a dicing operation of an active element, there is a limitation in the dicing operation. However, in the ultrasonic probe according to an embodiment, the active element can be diced by cutting the dicing buffer layer disposed on a rear surface of the active element. Therefore, channel separation of the active element is fabricated, and multi-dimensional probe can be manufactured.

In addition, in the ultrasonic probe according to an embodiment, each of the dicing buffer layer and the copper sheet constituting the sono-stair structure has a uniform thickness. Accordingly, the ultrasonic probe may generate periodic and uniform back-reflected waves. Moreover, the ultrasonic probe may be free from restrictions on thickness, material, shape, and the like of a support formed below the copper sheet.

Furthermore, in the ultrasonic probe according to an embodiment, the copper sheet is formed below the dicing buffer layer. Accordingly, since the copper sheet has high thermal conductivity to dissipate heat, overheating of the ultrasonic probe can be prevented.

### [Description of Drawings]

FIG. 1 is a view schematically illustrating a main configuration of an ultrasonic probe according to an embodiment of the present invention;
FIGS. 2 and 3 are views illustrating a detailed structure of an ultrasonic probe according to an embodiment of the present invention;
FIG. 4 is a block diagram illustrating a configuration of an ultrasonic diagnostic apparatus according to an embodiment of the present invention;
FIG. 5 is a view illustrating differences in configuration and performance between an ultrasonic probe of a conventional structure and an ultrasonic probe of a sono-stair structure according to an embodiment of the present invention; and
FIG. 6 is a view illustrating that an ultrasonic probe of a sono-stair structure according to an embodiment of the present invention is applicable to probes of various frequencies and shapes.

### [Mode of the Invention]

Hereinafter, embodiments of the present invention will be described in detail with reference to the accompanying drawings. In the following description of the present invention, if a detailed description of related known functions or configurations is determined to unnecessarily obscure the gist of the present invention, the detailed description thereof will be omitted herein. In addition, the terms described below are defined in consideration of the functions in the embodiments of the present invention, and these terms may be varied according to the intent or custom of a user or an operator. Therefore, the definitions of the terms used herein should follow contexts disclosed herein.

In this specification, when a first material layer is referred to as being "on" or "above" a second material layer, it should be interpreted to include not only the case where the first material layer is directly on or above the second material layer, but also the case where another third material layer is intervening between the first material layer and the second material layer, unless there is an explicit description excluding such an arrangement.

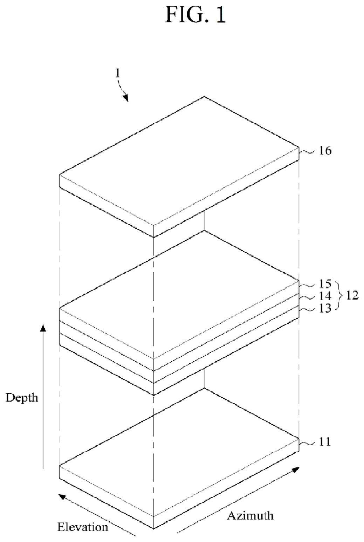

FIG. 1 is a diagram schematically illustrating a main configuration of an ultrasonic probe according to an embodiment of the present invention.

Hereinafter, it is specified that the term "schematically" means that the illustrated drawings show a relative positional relationship or a stacking relationship between components included in the ultrasonic probe. Accordingly, the specific shape, thickness, and the like of each of the components included in the ultrasonic probe may not necessarily coincide with those illustrated in the drawings.

Referring to FIG. 1, an ultrasonic probe 1 includes a support 11, a sono-stair layer 12, and an active element 16.

The active element 16 generates and transmits an ultrasonic signal to a target object, and receives an ultrasonic signal reflected from the target object. The active element 16 includes at least one piezoelectric element.

The active element 16 according to an embodiment has a thickness corresponding to 1/4 wavelength to 2/3 wavelength (1/4 λ to 2/3λ) based on the center frequency of the ultrasonic probe 1. Preferably, the active element 16 may have a thickness corresponding to 1/2 wavelength based on the center frequency of the ultrasonic probe 1.

When an active element having a thickness of less than or equal to a 1/4 wavelength based on the center frequency of the ultrasonic probe, there are limitations in processing the active element when the ultrasonic probe is used as a high-frequency probe. In addition, when a piezoelectric single crystal is applied, an allowable driving voltage is reduced to half due to a low coercive electric field.

In contrast, the active element 16 according to an embodiment has a thickness corresponding to 1/4 wavelength to 2/3 wavelength (1/4 λ to 2/3λ) based on the center frequency of the ultrasonic probe 1. Accordingly, the ultrasonic probe 1 is applicable to high frequencies. Furthermore, the ultrasonic probe 1 has a wide allowable range of driving voltage, fabrication of the dicing buffer layer 14 is easy, and manufacturing cost is low.

Depending on the number of piezoelectric elements in the active element 16, when there is a single piezoelectric element, the ultrasonic probe 1 is a single-element probe. In contrast, when a plurality of piezoelectric elements are provided, the ultrasonic probe 1 may be an array-type probe. Types of array-type probes include a linear array, a convex array, a phased array, and the like.

The ultrasonic probe 1 according to an embodiment is applicable to single-element probes and all types of array-type probes. In particular, the ultrasonic probe 1 may include a copper sheet 13, a dicing buffer layer 14, and a flexible printed circuit board (hereinafter referred to as an "FPCB") 15, which are arranged in a sono-stair structure between the active element 16 and the support 11. Hereinafter, the copper sheet 13, the dicing buffer layer 14, and the FPCB 15 will be referred to as a sono-stair layer 12.

The copper sheet 13, the dicing buffer layer 14, and the FPCB 15 are formed of materials having different impedances, and thus the ultrasonic probe 1 of the present invention may generate periodic and uniform back-reflected waves with respect to an ultrasonic signal generated in the active element 16 and transmitted toward the support 11.

Hereinafter, the structures of the copper sheet 13, the dicing buffer layer 14, and the FPCB 15 will be described in detail.

First, a dicing buffer layer 14 is formed below the active element 16 to facilitate channel separation during a dicing process of the active element 16.

In general, a backing layer is provided at a lowermost end of an ultrasonic probe, and an active element is provided on an upper end of the backing layer. In this case, when performing a process of dicing the active element, the backing layer needs to be cut, but the dicing operation of the active element is difficult due to powder contained in the backing layer. Accordingly, channel separation of the active element is not easily achieved.

However, according to an embodiment, the dicing buffer layer 14 is formed between the active element 16 and the support 11 having a backing layer function. In this case, during dicing of the active element 16, instead of cutting the backing layer, only the dicing buffer layer 14 needs to be cut, so that the channel separation of the active element 16 is facilitated. Accordingly, it is easy to manufacture a multi-dimensional probe.

As the dicing buffer layer 14 is formed between the copper sheet 13 and the FPCB 15, the dicing buffer layer 14 may prevent the copper sheet 13 and the FPCB 15 from being in direct contact with each other. To this end, the dicing buffer layer 14 may be made of a non-conductive material. For example, the dicing buffer layer 14 may be formed using an epoxy material. Since the epoxy material can provide an adhesive function, the copper sheet 13 and the FPCB 15 may be bonded to each other by the dicing buffer layer 14 implemented with the epoxy material.

In the above-described embodiment, a case in which the dicing buffer layer 14 is formed between the copper sheet 13 and the FPCB 15 has been described as an example; however the arrangement positions of the copper sheet 13, the FPCB 15, and the dicing buffer layer 14 constituting such a sono-stair layer 12 are not limited to the above-described example, and these components may be arranged in various positions.

In addition to the grind relief layer 140, at least one circuit layer may be formed between the copper sheet 13 and the FPCB 15.

The FPCB 15 formed between the active element 16 and the dicing buffer layer 14 enables electrical connection of a plurality of channels separated through the dicing operation of the active element 16.

The copper sheet 13 is formed below the dicing buffer layer 14. The copper sheet 13 may have high thermal conductivity and thus can dissipate heat, thereby preventing overheating of the ultrasonic probe 1. The copper sheet 13 may have a thickness of a 1/20 wavelength or more based on the center frequency of the ultrasonic probe 1.

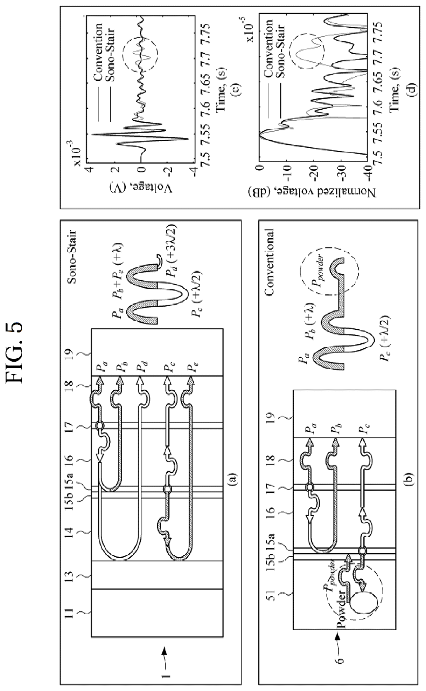

Due to the uniform thickness of each of the dicing buffer layer 14 and the copper sheet 13, the ultrasonic probe 1 may generate periodic and uniform back-reflected waves. Accordingly, the support 11 formed beneath the copper sheet 13 may be free from restrictions on thickness, material, shape, and the like. An embodiment in which the ultrasonic probe 1 generates periodic and uniform back3-reflected waves due to the uniform thickness of each of the dicing buffer layer 14 and the copper sheet 13 will be described below with reference to FIG. 5.

The support 11 minimizes the reflection of an ultrasonic signal generated from the active element 16 that propagates in an undesired direction, which is toward the support 11. As described above, due to the uniform thickness of each of the dicing buffer layer 14 and the copper sheet 13, the support 11 has no limitations in thickness, material, shape, and the like. Therefore, in the ultrasonic probe 1, it is easy to change the material, substance, shape, and the like of the support 11. For example, the ultrasonic probe 1 may increase a powder content in the support 11 to improve thermal conductivity, and may use aluminum as a material. Additionally, the ultrasonic probe 1 may include a metal material in the support 11 for heat dissipation.

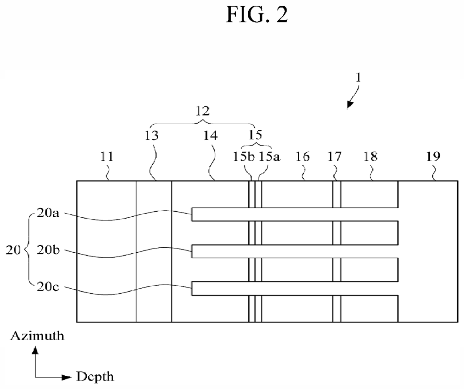

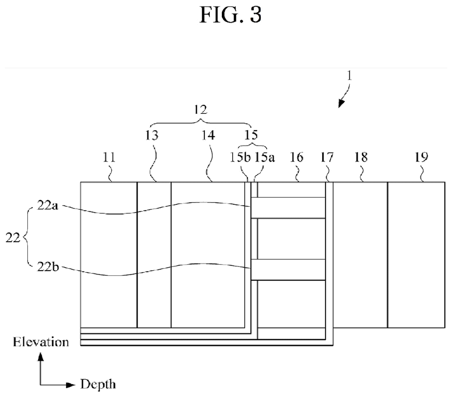

FIGS. 2 and 3 are views illustrating a detailed structure of an ultrasonic probe according to an embodiment of the present invention.

Referring to FIGS. 2 and 3, an ultrasonic probe 1 includes a support 11, a sono-stair layer 12, an active element 16, a ground sheet 17, a matching layer 18, an acoustic lens 19, a first kerf 20, and a second kerf 22.

Since the support 11, the sono-stair layer 12, and the active element 16 have been described above with reference to FIG. 1, the following description will focus on components not described with reference to FIG. 1.

The active element 16 according to an embodiment generates an ultrasonic signal when energy is applied, such as by applying a voltage to the FPCB 15 and the ground sheet 17 located at opposite ends thereof. The ultrasonic signal generated by the active element 16 according to an embodiment may have various frequencies. In particular, the ultrasonic signal may have a high frequency.

The FPCB 15 of the sono-stair layer 12 is formed between the active element 16 and the dicing buffer layer 14 for electrical connection of a plurality of separated channels of the active element 16.

The FPCB 15 may include a first FPCB 15a and a second FPCB 15b. The first FPCB 15a is formed between the active element 16 and the second FPCB 15b, and the second FPCB 15b is formed between the first FPCB 15a and the dicing buffer layer 14. The first FPCB 15a may include a metal, for example, copper (Cu), and the second FPCB 15b may include polyimide (PI).

The matching layer 18 is located on a front surface of the active element 16. The matching layer 18 matches acoustic impedance between the active element 16 and a target object, thereby transmitting an ultrasonic signal generated from the active element 16 to the target object or reducing a loss of a reflected ultrasonic signal returning from the target object. The matching layer 18 may also serve as a buffer that reduces problems such as image distortion caused by a sudden change in acoustic impedance between the active element 16 and the target object.

The matching layer 18 may include a plurality of layers each having a different acoustic impedance magnitude, the plurality of layers being formed using N materials (N is an integer greater than or equal to 2), to match acoustic impedance between the active element 16 and the target object. In this case, a variation range of acoustic impedance of the matching layer 18 is reduced, thereby improving bandwidth and sensitivity of the ultrasonic probe 1.

The ground sheet 17 may be formed between the matching layer 18 and the active element 16 according to an embodiment. Although not illustrated, the matching layer 18 may be arranged between the ground sheet 17 and the active element 16. In this case, the matching layer 18 may include an electrically conductive material, and thus the ground sheet 17 and the active element 16 may exchange electrical signals with each other through the matching layer 18. As described above, when the active element 16, the matching layer 18, and the ground sheet 17 are sequentially stacked, a two-dimensional (2D) array probe in which electrical connection is fabricated may be implemented.

The acoustic lens 19 is located at an outermost portion of the ultrasonic probe 1. An ultrasonic signal that has passed through the matching layer 18 may be directed toward the target object through the acoustic lens 19. The acoustic lens 19 according to an embodiment is configured such that the ultrasonic signal can be transmitted with no loss or with minimized loss, and is implemented to minimize loss due to reflection/re-reflection at an interface. In addition, the acoustic lens 19 may function to protect the matching layer 18 from wear. To this end, an acoustic impedance Zlens of the acoustic lens 19 may be identical or similar to an acoustic impedance of human soft tissue. For example, the acoustic lens 19 may include a material such as urethane, RTV silicone, or the like.

The ultrasonic probe 1 may include the first and second kerfs 20 and 22 that divide the active element 16 into a plurality of piezoelectric elements. The first and second kerfs 20 and 22 may be provided in one or more numbers. FIG. 2 illustrates a plurality of first kerfs 20 (20a, 20b, and 20c) that divide the active element 16 along a first direction (an azimuth direction), and FIG. 3 illustrates a plurality of second kerfs 22 (22a and 22b) that divide the active element 16 along a second direction (an elevation direction). The first and second kerfs 20 and 22 refer to spaces formed by a dicing process in which the active element 16 is divided into the plurality of piezoelectric elements, and the first and second kerfs 20 and 22 may be formed along directions intersecting each other.

The first and second kerfs 20 and 22 described above may be formed between the plurality of piezoelectric elements along a depth direction of the ultrasonic probe 1. The first and second kerfs 20 and 22 may have a structure filled with a material constituting the acoustic lens 19, but are not limited thereto.

At this time, the first and second kerfs 20 and 22 are formed between the plurality of piezoelectric elements along the first direction (azimuth direction) and the second direction (elevation direction) of the ultrasonic probe 1, and thus the ultrasonic probe 1 may have a multi-dimensional (multi-D) array structure that forms one or more focal points also in the second direction (elevation direction). In addition, although not illustrated, the ultrasonic probe 1 according to an embodiment of the present invention may have a 2D array structure in which a kerf is formed only in the first direction (azimuth direction).

The first kerf 20 may be formed extending from an upper surface of the matching layer 18 to a first position within the dicing buffer layer 14 through the ground sheet 17, the active element 16, and the FPCB 15. In addition, the second kerf 22 may be formed only in the active element 16 without being formed in the matching layer 18 and the ground sheet 17. FIG. 3 illustrates that the second kerf 22 is formed in the active element 16 and the first FPCB 15a.

The first FPCB 15a and the second FPCB 15b may extend not only between the active element 16 and the dicing buffer layer 14, but also to cover side surfaces of the dicing buffer layer 14, the copper sheet 13, and the support 11.

The ground sheet 17 may also extend not only between the active element 16 and the matching layer 18, but also to side surfaces of the active element 16, the first FPCB 15a, the second FPCB 15b, the dicing buffer layer 14, the copper sheet 13, and the support 11.

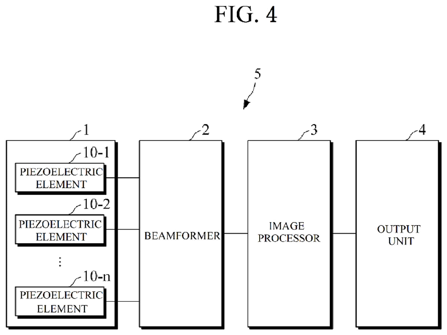

FIG. 4 is a block diagram illustrating a configuration of an ultrasonic diagnostic apparatus according to an embodiment of the present invention.

Referring to FIG. 4, an ultrasonic diagnostic apparatus 5 includes an ultrasonic probe 1, a beamformer 2, an image processor 3, and an output unit 4.

The ultrasonic probe 1 may include a plurality of piezoelectric elements 10-1, 10-2,..., and 10-n.

The beamformer 2 drives the ultrasonic probe 1 to transmit an ultrasonic signal to a target object and processes a reflected signal returning from the target object to generate a beam signal.

The image processor 3 receives the beam signal from the beamformer 2 and generates an ultrasonic image.

The output unit 4 displays the ultrasonic image generated through the image processor 3 to the outside.

FIG. 5 provides views and graphs illustrating differences in configuration and performance between an ultrasonic probe of a conventional structure and an ultrasonic probe of a sono-stair structure according to an embodiment of the present invention.

More specifically, (a) illustrates a configurationof an ultrasonic probe having a sono-stair structure and a form of a back-reflected wave according to an embodiment of the present invention, and (b) illustrates a configuration of an ultrasonic probe having a conventional structure and a form of a back-reflected wave. (c) is a graph comparing voltage magnitudes over time between (a) and (b), and (d) is a graph comparing magnitudes of normalized voltages over time between (a) and (b).

In interpreting (c) and (d), it should be noted that the support 11 was tested in the same state as a backing layer condition of the ultrasonic probe of the conventional structure, that is, in a condition including agglomerated powder.

Referring to (a) of FIG. 5, an ultrasonic probe 1 of a sono-stair structure includes a support 11, a copper sheet 13, a dicing buffer layer 14, a second FPCB 15b, a first FPCB 15a, an active element 16, a ground sheet 17, a matching layer 18, and an acoustic lens 19.

In contrast, an ultrasonic probe 6 of a conventional structure as shown in (b) of FIG. 5 includes a backing layer 51, a second FPCB 15b, a first FPCB 15a, an active element 16, a ground sheet 17, a matching layer 18, and an acoustic lens 19.

Compared to the ultrasonic probe 6 of the conventional structure, the ultrasonic probe 1 of the sono-stair structure has a configuration in which the dicing buffer layer 14 and the copper sheet 13 are arranged between the active element 16 and the support 11, and the ultrasonic probe 1 of the sono-stair structure has a structure in which the support 11 is formed instead of the backing layer 51 disclosed in (b) of FIG. 5. Furthermore, in the ultrasonic probe 1 of the sono-stair structure, the dicing buffer layer 14 and the copper sheet 13 each have a uniform thickness. For example, the dicing buffer layer 14 has a thickness corresponding to 1/5 wavelength to 1/4 wavelength based on the center frequency of the ultrasonic probe 1, and the copper sheet 13 has a thickness corresponding to 1/20 wavelength or more based on the center frequency of the ultrasonic probe 1.

Accordingly, the ultrasonic probe 1 of the sono-stair structure may generate a periodic and uniform back-reflected wave. Furthermore, the support 11 formed beneath the copper sheet 13 may be free from restrictions on thickness, material, shape, and the like.

As shown in (c), it can be confirmed that the ultrasonic probe 1 of the sono-stair structure has a smaller voltage variation over the same time period compared to the ultrasonic probe 6 of the conventional structure.

As shown in (b), in the ultrasonic probe 6 of the conventional structure, an error may appear in an ultrasonic image through signal processing in an imaging system due to a sound wave P_{powder} reflected from powder contained in the backing layer 51. On the other hand, since the ultrasonic probe 1 of the sono-stair structure can block nonuniform reflected waves generated from the rear surface, the voltage variation is small as shown in (c).

In addition, as shown in (d), it can be confirmed that the ultrasonic probe 1 of the sono-stair structure has a larger voltage over the same time period compared to the ultrasonic probe 6 of the conventional structure. In the case of an organ or lesion that is small in size or has a small difference in acoustic impedance, it is not easy to detect a signal reflected after applying ultrasound to the lesion.

However, since the ultrasonic probe 1 of the sono-stair structure has a high voltage, transmission and reception sensitivity is increased, and thus even a minute signal can be detected. Therefore, the ultrasonic probe 1 of the sono-stair structure can accurately represent, in an ultrasonic image, an organ or lesion that is small in size or has a small difference in acoustic impedance.

Sound waves generated by the active element 16 will be described in detail as follows.

As shown in (a) and (b), when a driving voltage is applied to the active element 16, sound waves are generated at upper and lower surfaces of the active element 16, and then main sound waves Pₐ, P_{c}, and P_{b} propagating toward the matching layer 18 are formed.

In the probe 6 of the conventional structure as shown in (b), sound waves traveling toward the backing layer 51 are all canceled out by attenuation of a rear layer.

However, in the probe 1 of the sono-stair structure as shown in (a), sound waves P_{d} and Pₑ generated by the active element 16 and traveling toward the copper sheet 13 are reflected by the copper sheet 13 and cause constructive interference with the main sound waves Pₐ, P_{c}, and P_{b}, thereby generating an amplified composite wave Pₐ, P_{c}, P_{b}, P_{d}, and Pₑ.

In addition, as shown in (b), in the probe 6 of the conventional structure, a sound wave P_{Powder} is irregularly generated due to unintended impurities in the backing layer 51, whereas, in the probe 1 of the sono-stair structure of (a), it can be confirmed that the sound waves P_{d} and Pₑ are mostly reflected by the copper sheet 13 and are not affected by the support 11. Accordingly, in the ultrasonic probe 1 according to an embodiment of the present invention, there are fewer restrictions on the thickness, material, shape, and the like of the support 11, and thus the support 11 may be formed to include a metal material to improve heat dissipation characteristics.

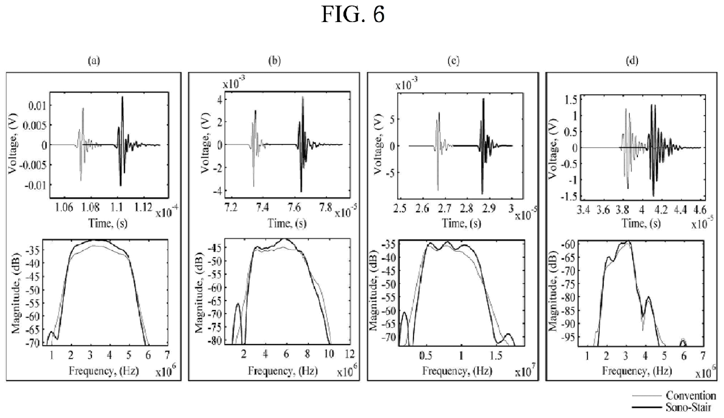

FIG. 6 is a view illustrating that an ultrasonic probe of a sono-stair structure according to an embodiment of the present invention is applicable to probes of various frequencies and shapes.

More specifically, FIG. 6 illustrates changes in voltage magnitude over time and changes in normalized magnitude over time in environments of probes a, b, c, and d having various frequencies and shapes.

Referring to FIGS. 1, 5 and 6, the ultrasonic probe 1 of the sono-stair structure according to an embodiment is applicable to probes of various frequencies and shapes. For example, it can be confirmed that the ultrasonic probe 1 of the sono-stair structure has a larger normalized voltage magnitude compared to the ultrasonic probe 6 of the conventional structure. Accordingly, the bandwidth of the ultrasonic probe 1 of the sono-stair structure is widened, and sensitivity is improved.

(a), (b), and (c) of FIG. 6 show results of finite element analysis, and are graphs obtained when the sono-stair structure is applied to a convex array probe, a phased array probe, and a linear array probe each having a different center frequency, from which it can be confirmed that an output voltage is increased and bandwidth is widened.

The center frequency of the convex array probe shown in (a) of FIG. 6 is 3 MHz, the center frequency of the phased array probe shown in (b) of FIG. 6 is 6 MHz, and the center frequency of the linear array probe shown in (c) of FIG. 6 is 9 MHz.

Frequency and wavelength have an inversely proportional relationship, and thicknesses of the active element 16, the dicing buffer layer 14, and the copper sheet 13 of (a) of FIG. 5 are changed based on a wavelength that varies according to the frequency. Accordingly, back-reflected waves P_{d} and Pₑ are coupled to the main sound waves Pₐ, P_{c}, and P_{b} with a constant time interval therebetween, so that an enhanced composite wave can be generated. As shown in the time-domain waveforms of FIG. 6, the output voltage of the ultrasonic probe 1 of the sono-stair structure is larger than the output voltage of the ultrasonic probe 6 of the conventional structure. In addition, as shown in the frequency spectrum of FIG. 6, various frequency components are enhanced, thereby widening the bandwidth.

(d) of FIG. 6 shows measurement results obtained by applying a sono-stair structure to a therapeutic 2D array probe in which a plurality of channels are formed in a narrow area, from which it can be confirmed that sound pressure is increased. Through this, regardless of the frequency and shape of the ultrasonic probe 1, the sono-stair structure is effective in improving the performance of the ultrasonic probe 1.

Also, in the ultrasonic probe 1 of the sono-stair structure, due to the uniform thickness of each of the dicing buffer layer 14 and the copper sheet 13, the support 11 is not limited in thickness, material, shape, and the like. Therefore, it is easy to change the material, substance, shape and the like of the support 11. For example, the ultrasonic probe 1 of the sono-stair structure may increase a powder content in the support 11 to improve thermal conductivity, and may use aluminum as a material. The support 11 may include a metal material for heat dissipation.

Furthermore, in the ultrasonic probe 1 of the sono-stair structure, the copper sheet 13 having high thermal conductivity is stacked below the dicing buffer layer 14. Accordingly, heat can be effectively dissipated, thereby preventing overheating of the ultrasonic probe 1.

The present invention has been described above with reference to embodiments thereof. Those skilled in the art to which the present invention pertains will understand that the present invention may be implemented in a modified form without departing from the essential characteristics of the present invention. Therefore, the disclosed embodiments should be considered in a descriptive sense rather than a restrictive sense. The scope of the present invention is set forth in the claims rather than the foregoing description, and all differences within the scope equivalent thereto should be construed as being included in the present invention.

## Claims

1. An ultrasonic probe comprising:
an active element having a thickness corresponding to 1/4 wavelength to 2/3 wavelength based on a center frequency of the ultrasonic probe;
a matching layer formed above the active element;
a sono-stair layer comprising a dicing buffer layer formed below the active element to separate channels during a dicing process of the active element, and a copper sheet formed below the dicing buffer layer; and
a support formed below the sono-stair layer.

2. The ultrasonic probe of claim 1, wherein the active element has a thickness corresponding to 1/2 wavelength based on a center frequency of the ultrasonic probe.

3. The ultrasonic probe of claim 1, wherein the dicing buffer layer has a thickness corresponding to 1/5 wavelength to 1/4 wavelength based on a center frequency of the ultrasonic probe.

4. The ultrasonic probe of claim 3, wherein the dicing buffer layer has a thickness corresponding to 1/4 wavelength based on the center frequency of the ultrasonic probe.

5. The ultrasonic probe of claim 1, wherein the dicing buffer layer is made of an epoxy material.

6. The ultrasonic probe of claim 1, wherein the copper sheet has a thickness corresponding to 1/20 wavelength or more based on a center frequency of the ultrasonic probe.

7. The ultrasonic probe of claim 1, wherein the sono-stair layer further comprises a flexible printed circuit board formed between the active element and the dicing buffer layer for electrical connection of a plurality of separated channels of the active element.

8. The ultrasonic probe of claim 7, wherein the flexible printed circuit board comprises:
a first flexible printed circuit board formed between the active element and a second flexible printed circuit board; and
the second flexible printed circuit board formed between the first flexible printed circuit board and the dicing buffer layer.

9. The ultrasonic probe of claim 8, wherein the first flexible printed circuit board includes copper (Cu), and the second flexible printed circuit board includes polyimide (PI).

10. The ultrasonic probe of claim 1, further comprising a ground sheet formed between the active element and the matching layer.

11. The ultrasonic probe of claim 1, further comprising a first kerf formed extending from an upper surface of the matching layer through the active element to a first position within the dicing buffer layer.

12. The ultrasonic probe of claim 1, further comprising a second kerf formed only in the active element,
wherein the second kerf is formed along a direction intersecting the first kerf.

13. The ultrasonic probe of claim 1, wherein the matching layer comprises a plurality of layers each having a different acoustic impedance magnitude, the plurality of layers being formed using N materials (N is an integer greater than or equal to 2), to match acoustic impedance between the active element and a target object.
